# EUROPEAN PATENT SPECIFICATION

(11) **EP 3 741 218 B1**
(45) Date of publication and mention of the grant of the patent: **14.05.2025**
(21) Application number: 19382407.5
(22) Date of filing: 21.05.2019
(51) Int. Cl.: A23K 10/16, A23K 10/18, A23K 50/30, A61K 35/747, A61P 1/12, C12N 1/20, C12R 1/225

(54) **LACTOBACILLUS SALIVARUS STRAIN AND ITS USE IN THE PREVENTION AND TREATMENT OF POST-WEANING DIARRHEA IN PIGLETS**
LACTOBACILLUS-SALIVARUS-STAMM UND DESSEN VERWENDUNG ZUR VORBEUGUNG UND BEHANDLUNG VON DURCHFALL NACH DEM ABSTILLEN IN FERKELN
SOUCHE DE LACTOBACILLUS SALIVARUS ET SON UTILISATION DANS LA PRÉVENTION ET LE TRAITEMENT DE LA DIARRHÉE POST-SEVRAGE CHEZ LES PORCELETS

(43) Date of publication of application: 25.11.2020
(73) Proprietor: Probisearch, S.L.U., 28760 Tres Cantos, Madrid (ES)
(72) Inventor: DE ANDRÉS LEO, Javier, E-28760 Tres Cantos, Madrid (ES); ESPINOSA MARTOS, Irene, E-28760 Tres Cantos, Madrid (ES); GARCÍA CARRAL, Cristina, E-28760 Tres Cantos, Madrid (ES); JIMÉNEZ QUINTANA, Esther Antonia, E-28760 Tres Cantos, Madrid (ES)
(74) Representative: ABG Intellectual Property Law, S.L.

(56) References cited:
- KR-A- 20030 063 960
- KR-B1- 100 585 392
- KR-B1- 101 335 455
- YEO SOYOUNG ET AL: "Development of putative probiotics as feed additives: validation in a porcine-specific gastrointestinal tract model", APPLIED MICROBIOLOGY AND BIOTECHNOLOGY, SPRINGER BERLIN HEIDELBERG, BERLIN/HEIDELBERG, vol. 100, no. 23, 15 September 2016 (2016-09-15), pages 10043 - 10054, XP036094403, ISSN: 0175-7598, [retrieved on 20160915], DOI: 10.1007/S00253-016-7812-1
- WU YUNPENG ET AL: "Protective effects ofLactobacillus plantarumon epithelial barrier disruption caused by enterotoxigenicEscherichia coliin intestinal porcine epithelial cells", VETERINARY IMMUNOLOGY AND IMMUNOPATHOLOGY, ELSEVIER BV, AMSTERDAM, NL, vol. 172, 5 March 2016 (2016-03-05), pages 55 - 63, XP029481577, ISSN: 0165-2427, DOI: 10.1016/J.VETIMM.2016.03.005
- DAVID H FRANCIS: "Enterotoxigenic Escherichia coli infection in pigs and its diagnosis", J SWINE HEALTH PROD, vol. 10, no. 4, 2002, pages 171 - 175, XP055638918, DOI: https://www.aasv.org/shap/issues/v10n4/v10n4p171.html
- SCHILLING ANDREW T: "The Effect of pH on the Bacterium E. coli", CALIFORNIA STATE SCIENCE FAIR, 1 January 2008 (2008-01-01), XP093170045, Retrieved from the Internet <URL:https://csef.usc.edu/History/2008/Projects/J1429.pdf> [retrieved on 20240604]
- SNOECK VEERLE ET AL: "Influence of porcine intestinal pH and gastric digestion on antigenicity of F4 fimbriae for oral immunisation", VETERINARY MICROBIOLOGY, vol. 98, no. 1, 1 January 2004 (2004-01-01), NL, pages 45 - 53, XP093170033, ISSN: 0378-1135, DOI: 10.1016/j.vetmic.2003.10.020

## Description

### FIELD OF THE INVENTION

The invention relates to the field of probiotic strains, in particular to a new probiotic strain of *Lactobacillus salivarius,* to compositions comprising said probiotic strain, and its use for the prevention of diseases, in particular of post-weaning diarrhea in pigs.

### BACKGROUND OF THE INVENTION

Reduction of the lactation period has been an important factor in improving productivity in the swine industry. However, early weaning entails several problems since the piglet's digestive apparatus is undeveloped and low feed intake and diarrhea are common.

Diarrhea (or scour) in piglets is one of the most common problems in pig farming affecting hundreds of millions of piglets born globally each year. It can result in reduced weight gain, high cost of treatment and frequent mortality. Piglets set back in health at an early age, tending to remain at a weight and performance disadvantage in later life. Therefore, diarrhea not only has a detrimental impact on piglet health, but on farm profitability.

Piglet diarrhea caused by *Escherichia coli* (*E. coli*) strains is common within the first weeks after weaning, and also in the first weeks of life. Diarrhea in pre-weaned piglets (suckling pigs) can be fatal since piglets can become quickly dehydrated and mortality rates are high.

Weaning is a critical period in a piglet's life. It must cope with separation from the sow, the transition from highly digestible milk to a less digestible and more complex solid feed, a new environment, movement and separation from littermates, and exposure to unfamiliar pigs. These stress factors can lead to reduced feed intake and reduced piglet growth. Additionally, the newly-weaned pigs' immune and digestive systems are still maturing, making the piglet more susceptible to antigenic challenges (nutritional or microbial), which can lead to inflammatory responses. Inflammation induces a negative impact on the digestive and absorptive capabilities of the gut, and overall gut health creating an opportunity for an animal to become more susceptible to pathogens. An animal whose reduced feed intake is poor at the time of pathogen exposure will become sick. The absence of feed in a piglet's gut results in a microbial imbalance, leading to higher occurrences of diseases. During this period, post-weaning diarrhea (PWD) is very frequent. PWD generally affects pigs during the first two weeks after weaning and is characterized by sudden death or diarrhea, dehydration and growth retardation in surviving piglets. PWD is commonly associated with the proliferation of *E. coli* in the pig intestine, typically belonging to serogroups 0149, 08, 0138, 0139, 0141, 0147, and 0157. Feed intake is usually reduced initially after weaning and the pig may develop anorexia of variable duration and extent between farms, depending on livestock management and the nature of the feed. Underfeeding during weaning reduces growth performance of pigs and contributes to intestinal inflammation and adversely affects villous height and crypt depth. This morphological disruption of the intestinal mucosa promotes the creation of an ideal environment for the multiplication of bacteria such as *E. coli* and allows toxins and bacteria to cross the epithelium as a result of this inflammation.

Antibiotics, such as colistin, are widely used for the control of PWD in pigs. However, in humans this antibiotic is considered as one of the last therapeutic options for the treatment of infections caused by multidrug-resistant Gram-negative bacteria. Since food producing animals, and in particular pigs, have been singled out as the most potential reservoirs for spread and amplification of colistin resistance, scientists and regulatory agencies have recommended reducing the use of said antibiotic in animal production.

Other strategies used in pigs for controlling PWD are feed supplements such as zinc oxide, organic acids (such as citric, fumaric, lactic, propionic, benzoic and formic acids), prebiotics (such as inulin, oligosaccharides, e.g. galactooligosaccharides and fructooligosaccharides), probiotics (such as *L. rhamnosus* and *L. acidophilus*)*,* dehydrated porcine plasma, antimicrobial peptides (such as lactoferrin, cecropin, defensing, plectasin and bacteriocins), specific egg yolk antibodies, bacteriophages and probiotics.

Probiotics are defined by the World Health Organization as live microorganisms that, when administered in adequate amounts, confer a health benefit on the host. Probiotic products may be formulated as capsules, tablets, powders (which are regulated as a dietary supplement), and a feed or food ingredient (e.g., yogurts, kefirs), or as a drug. Probiotics may exert a wide range of beneficial effects, such as balancing the host gut microbiota and interacting with the innate and adaptive immune system, which may promote resistance against pathogens. The most commonly used probiotics are *Lactobacillus* and *Bifidobacterium* species, followed by the genera *Streptococcus, Enterococcus, Propionibacterium, Bacillus,* and *Escherichia coli.* In addition, some yeast species are used as probiotics, for example, *Saccharomyces boulardii* and *Saccharomyces cerevisiae* are frequently used to treat gastrointestinal disorders.

*Lactobacillus salivarius* is a species commonly isolated from the gastrointestinal tract of humans and animals with potential probiotic properties. Results of previous studies have shown a considerable functional diversity existing both in the chromosome and in the plasmids of different strains. A variety of bacteriocins with different spectra of activity against pathogens are coded in these plasmids [Harris et al., Microb Genom., 2017, 3, e000115]. The production of these antibacterial proteins is widely distributed within the strains of this species, providing them with the ability to modulate the intestinal microbiota and reduce the impact of pathogens, such as *Listeria monocytogenes* [O 'Shea et al., Gut Microbes, 2012, 3(5), 468-473]. Other strains of *L. salivarius* have shown activity against *Streptococcus mutans,* which is involved in the production of caries [Sañudo et al., Arch Oral Biol., 2017, 84, 58-63], and against *Aggregatibacter actinomycetemcomitans,* which is involved in periodontal disease [Sajedinejad et al., Probiotics Antimicrob Proteins, 2018, 10, 485-495]. At vaginal level, strains of *L. salivarius* present antimicrobial activity against *Gardnerella vaginalis* and *Candida albicans,* which are implicated in vulvovaginitis [Kang et al., Probiotics Antimicrob Proteins, 2018, 10, 343-349]. Other *L. salivarius* strains have shown activity against *Staphylococcus aureus* [Heikkilä and Saris, J Appl Microbial., 2003, 95, 471-478; Kang et al., Pathog Dis., 2017, 75(2). doi:10.1093/femspd/ftx009], *S. epidermidis* [Arroyo et al., Clin Infect Dis., 2010, 50, 1551-1558] and against enterobacteria [Abhisingha et al., Probiotics Antimicrob Proteins, 2018, 10, 218-227].

In animals, *L. salivarius* has been effective in the control of *Salmonella, Brachyspira pilosicoli* and *Campylobacter jejuni* in poultry farms [Mappley et al., Appl Environ Microbiol., 2011, 77, 5402-5411; Ghareeb et al., Poult Sci., 2012, 91, 1825-1832; Yamakazi et al., Br Poult Sci., 2012, 53, 183-189]. In pigs, the administration of *L. salivarius* together with *Bacillus subtilis* has improved the immune status of the intestinal mucosa of the piglets, which is a reinforcing measure to avoid post-weaning diarrhea [Zhang et al., Curr Microbiol., 2011, 62, 1623-1631; and Deng et al., Res Vet Sci., 2013, 94, 62-68]. Despite these positive results, interaction of *Lactobacillus* with pre-existent microbiota may also have negative effects, as evidenced by the study conducted by Trevisi et al. [Animal, 2011, 9(9), 1354-1360]. *Lactobacillus rhamnosus GG* did not only fail to prevent or reduce the detrimental effect of *E. coli* infection on the growth performance and health statues of weaned piglets, but also interacted negatively with the intestinal barrier. YEO SOYOUNG ET AL, Applied Microbiology and Biotechnology, Vol. 100, No. 23, 15, pages 10043-10054, disclosing Lactobacillus salivarius LS6, which is capable of preventing infection with the enterotoxigenic E. coli strain K88. KR101335455B1 discloses acid resistant, bile resistant Lactobacillus salivarius PL9028 (KACC91678P). KR20030063960A discloses acid- and bile-tolerant Lactobacillus salivarius Probio-37 (KCCM-10328).

In summary, post-weaning diarrhea (PWD) is a serious threat for the swine industry worldwide. Alternatives to antibiotic treatments are necessary to avoid transfer of antibiotic resistance to humans. Thus, there remains a need for new treatments of diarrhea in piglets, in particular of PWD.

### BRIEF DESCRIPTION OF THE INVENTION

The inventors have isolated and characterized a probiotic strain of *Lactobacillus salivarius* (PS21603) isolated from the intestine of a wild boar, which has surprisingly been found to reduce the fecal *E. coli* concentration, to be useful for the prevention of post-weaning diarrhea in piglets.

As shown in the examples, the *Lactobacillus salivarius* strain PS21603 of the invention has a great inhibitory activity of *Escherichia coli,* said *Lactobacillus salivarius* PS21603 strain has not shown any resistance to antibiotics, and animals treated with said strain have shown improved intestinal morphology (ratio of villous height to crypt depth and length of the small and large intestine). Therefore, the *Lactobacillus salivarius* strain PS21603 of the invention has shown a high potential for the prevention of PWD in pigs. The *Lactobacillus salivarius* PS21603 strain has been deposited at the Colección Española de Cultivos Tipo (CECT) and has been given accession number CECT 9673.

Thus, in one aspect, the present invention is directed to a strain of *Lactobacillus salivarius* PS21603.

In another aspect, the invention also relates to a composition comprising the strain as defined above.

In further aspect, the invention also relates to the strain or composition as defined above for use as a probiotic or for use as a medicament.

In another aspect, the invention is directed to the strain or the composition as defined above, for use in the prevention of post-weaning diarrhea (PWD) caused by an *E. coli* infection in piglets.

### FIGURES

Figure 1 shows the growth curve of the *L. salivarius* PS21603, represented as OD₆₀₀ variation of the OD₆₀₀ (optical density measured at 600 nm) over time (h). S is the standard error and R is the correlation coefficient.
Figure 2 represents the correlation between the OD₆₀₀ and the *L. salivarius* PS21603 concentration (expressed as log(CFU/mL). S is the standard error and R is the correlation coefficient.
Figure 3 shows the evolution of pH of the *L. salivarius* PS21603 growth medium with time (h). S is the standard error and R is the correlation coefficient.
Figure 4 represents the correlation of *L. salivarius* PS21603 concentration (log (CFU/mL)) with acid production (pH). S is the standard error and R is the correlation coefficient.
Figure 5 shows the evolution of OD₆₀₀ over time (h) for *L. salivarius* PS21603 at different pH.
Figure 6 shows the evolution of OD₆₀₀ over time (h) for *L. salivarius* PS21603 at different NaCl concentrations.
Figure 7 represents the bacterial concentration (log (CFU/mL)) of *L. salivarius* PS21603 and *E. coli* CECT501 at 0 h and 24 h of co-culture of both strains.

### DETAILED DESCRIPTION OF THE INVENTION

In the present invention, the term "probiotic" means a microorganism that exerts beneficial effects on the health of the host. It can be a live microbial fed supplement or medicament that beneficially affects the host by improving its microbial balance, a microbial preparation that contains live or dead bacteria, or a combination of both. Hosts suitable in the context of the invention includes any mammal, such as pigs, horses, cows, goats, ewes, dogs, cats, rats, mice and primates, such as human beings; more preferably pigs.

The term "pig" or "swine" includes domestic pigs and wild boars of any age, preferably piglets and weaners, both males and females. In particular, "piglets" refers to young pigs, such as from birth until weaning, which in pig farming generally takes place at the age of 2-4 weeks; and "weaners" refers to pigs from weaning until the age of about 10 weeks.

### Lactobacillus salivarius strain PS21603

The strain of *Lactobacillus salivarius* according to the invention is referred to as *Lactobacillus salivarius* PS21603 or *L. salivarius* PS21603. The strain *L. salivarius* PS21603 has been deposited by Probisearch SLU, Calle Santiago Grisolia 2, Tres Cantos, Spain in accordance with the Budapest Treaty on the International Recognition of the Deposit of Microorganisms for the Purpose of Patent Procedure at the Colección Española de Cultivos Tipo (CECT) on 5 June 2018 and has been given Accession Number CECT 9673.

The term "strain" is well-known in the field and means a genetic variant or subtype of a microorganism. A "variant" or "mutant" of a strain refer to any naturally-occurring or specifically developed strain obtained from the reference strain *L. salivarius* PS21603, mainly by mutation, that maintains or enhances the properties of the reference strain. Thus, in the present case, the invention discloses variants of the reference strain (which are not encompassed by the claims) in which one or more of the following properties associated to the *L. salivarius* PS21603 strain is preserved or enhanced:
- prevent or reduce scours in piglets or weaners,
- increase piglet or weaner weight,
- reduce piglet or weaner fecal *E. coli* concentration,
- reduce *E. coli* concentration in the 24-hour co-culture with the *E. coli* strain having accession number CECT 501 (given by the Colección Española de Cultivos Tipo), with serotype O149: K91, K88a, c: H10,
- increase the length of the small and/or large intestine of piglets or weaners, and
- improve the ratio of villous height to crypt depth of the intestine of piglets or weaners.

As used herein, the reference to that the variant strain maintains the properties of the reference strain *L. salivarius* PS21603 means that, while not showing an activity of 100% of the activity of the reference strain *L. salivarius* PS21603 from which it derives, the property is substantially preserved so that the variant shows at least 99%, at least 98%, at least 97%, at least 96%, at least 95%, at least 94%, at least 93%, at least 92%, at least 91%, at least 90%, at least 80%, at least 70%, at least 60%, at least 50% of the activity of the *L. salivarius* PS21603 strain from which it derives.

The person skilled in the art will decide upon the adequate method to be employed for determining each of the properties mentioned above for the strains, in particular using the methods described in the examples of the present application.

The present invention discloses strains of *L. salivarius,* that are not encompassed by the claims, having at least 95 % identity with the 16S rRNA sequence of the *L. salivarius* PS21603 strain, which is described in the examples [reference Stackebrandt & Goebel, 1994: "Taxonomic Note: A Place for DNA-DNA Reassociation and 16s rRNA Sequence Analysis in the Present Species Definition in Bacteriology" Int. J. Syst. Bacteriol. 44:846-849].

In a non-claimed embodiment, the strain according to the present invention has at least 97 % identity with the 16S rRNA sequence of the *L. salivarius* PS21603 strain, more preferably at least 98% identity, more preferably at least 99% identity. The strain according to the present invention has 100% identity with the 16S rRNA sequence of the *L. salivarius* PS21603 strain, i.e. the strain according to the present invention is the *Lactobacillus salivarius* PS21603 strain.

The strains of the invention, as demonstrated in the examples, have sufficient resistance to the conditions of the acidic microenvironment of the gastrointestinal tract so that said strains may survive passage through the gastrointestinal tract. The strains have also shown good resistance to osmotic stress, which allows them to withstand lyophilization-rehydration processes used in industrial production of probiotics.

The strain of the present invention may be alive or inactivated. Alive microorganisms produce a complete array of antigens, reproduce to increase the number of such organisms in the intestinal environment to better stimulate an immune response. Thus, in a preferred embodiment, the strain of the invention is alive.

As used herein, the term "inactivated" refers to a dead or inactivated cell of a microorganism which is no longer capable to form a single colony on a plate having medium specific for said microorganism, and it also encompasses lysates, fractions or extracts of the microorganism. The inactivation of a cell refers to a process of transforming a cell from viable to non-viable. The terms "viable", "alive" or "viability", as used herein, refer to the ability of a cell to maintain itself or recover its potentialities and survive until they are able to divide. Thus, a cell that is viable or alive indicates that the cell is able to survive and divide; conversely, a cell that is non-viable indicates that the cell is not able to survive and divide. It will be appreciated that non-viable cells include dead cells.

Viability can be determined by a number of assays that are conventional in the art. These include cytolysis or membrane leakage assays, such as the lactate dehydrogenase assay, the propidium iodide assay, the trypan blue assay and the 7-aminoactinomycin D assay, as well as genomic and proteomic assays that test the activation of stress pathways using DNA microarrays and protein chips. Viability can also be determined by checking the absence of cells after their culture in an appropriate culture medium.

### Compositions

In another aspect, the present invention relates to a composition comprising the *L. salivarius* PS21603 strain of the invention.

Preferably, the compositions of the invention comprises the *L. salivarius* PS21603 strain of the invention and a physiologically acceptable carrier or excipient and/or further ingredients as described further below. Preferably, the *L. salivarius* strain according to the invention is present in freeze-dried form.

The term "composition", as used in the present invention relates to any composition of matter comprising the strain of the invention, i.e. *L. salivarius* PS21603 strain.

In a preferred embodiment, the composition of the invention comprises the *L. salivarius* PS21603 strain or and a veterinary or pharmaceutically acceptable excipients. These compositions can also be named as "veterinary composition", "veterinary product", "pharmaceutical composition" or "pharmaceutical product".

As used in the present invention, the expressions "veterinary" and "pharmaceutical" refer to the same type of product or ingredient but the first one being intended for animal use and the second one being intended for human use.

As used in the present invention, the expressions "veterinary composition" or "veterinary product" are used herein interchangeably and refer to a formulation which has been adapted to administer a predetermined dose of one or several therapeutically useful agents, i.e. the strains of the invention, to a mammal animal, preferably pigs, in which a direct or indirect therapeutic effect of the composition is sought out. The veterinary composition of the invention contains a therapeutically effective amount of the *Lactobacillus salivarius* PS21603 strain of the invention, which is understood herein as an amount capable of providing a therapeutic effect, and which can be determined by the person skilled in the art by commonly used means.

As used in the present invention, the expressions "pharmaceutical composition" or "pharmaceutical product" are used herein interchangeably and refer to a formulation which has been adapted to administer a predetermined dose of one or several therapeutically useful agents to a mammal, preferably a piglet, in which a direct or indirect therapeutic effect of the composition is sought out. The pharmaceutical composition of the invention contains a therapeutically effective amount of the *Lactobacillus salivarius* PS21603 strain of the invention, which is understood herein as an amount capable of providing a therapeutic effect, and which can be determined by the person skilled in the art by commonly used means.

The "veterinary acceptable excipient" and "pharmaceutical acceptable excipient" refer to a diluent or carrier with which the active ingredient, i.e. the strains of the invention, is administered. Acceptable excipients for therapeutic use are well known in the veterinary and pharmaceutical art, and are described, for example, in Remington: The Science and Practice of Pharmacy, Lippincott Williams and Wilkins (22nd Ed., 2012). The choice of veterinary and pharmaceutical excipient can be selected with regard to the intended route of administration and standard pharmaceutical and veterinary practice. For example, such pharmaceutical and veterinary excipients include, but are not limited to, water, glucose, lactose, sucrose, mannitol, maltodextrin, (resistant) starch, cellulose or cellulose derivatives, e.g. methylcellulose, magnesium stearate, stearic acid, sodium saccharin, talcum, magnesium carbonate and the like; preferably maltodextrin. Water or aqueous solutions of saline solution and aqueous dextrose and glycerol solutions are preferably used as excipients. The pharmaceutical and veterinary compositions of the invention may comprise as - or in addition to - the excipients mentioned above any suitable binder(s), lubricant(s), suspending agent(s), coating agent(s), solubilizing agent(s). Preservatives, stabilizers, dyes and even flavoring agents may be provided in the pharmaceutical and veterinary compositions. Examples of preservatives include sodium benzoate, sorbic acid and esters of p-hydroxybenzoic acid. Antioxidants and suspending agents may also be used. There may be different composition/formulation requirements depending on the different delivery systems.

In a preferred embodiment, the composition of the invention comprises maltodextrin as the veterinary or pharmaceutically acceptable excipient.

In a particular embodiment the strain of the invention is present in a foodstuff. These compositions can also be named as terms "feed product", "feed composition", "food product" or "food composition". These compositions relate to a feed or food that beneficially affects one or more functions of the body, so as to provide better health and wellness. Accordingly, such a feed or food product may be intended for the prevention and/or treatment of a disease or a disease causing factor. Therefore, these compositions can also be named as functional feed or food for particular nutritional purposes, or veterinary feed.

As used in the present invention, the expressions "feed" and "food" and refer to a foodstuff but the first one being intended for animal use and the second one being intended for human use. In particular, the term "feed" refers to a commercial feed which is formulated according to the specific requirements of the target animal.

Non-limiting examples of suitable foodstuffs which can be used in the present invention are animal feed, including feed premix, final feed product, pellets, cereals (such as corn, oats and barley), fermented cereal based products, other cereal based powders, soybean, copra, straw, grass, sugar beet waste, fish meal, meat and bone meal, seeds, bread, cakes, semi- or synthetic diet formulations, infant formulae, clinical nutrition formulae, flours. Preferably the foodstuff is an animal feed, such as pig feed.

The effective amount of colony forming units (CFU) for each strain in the composition of the invention will be determined by the skilled person and will depend upon the final formulation and the times per day it will be administered. Preferably, the strain of the invention is present in the compositions of the invention in an amount that provides about 10⁵ CFU/day to about 10¹¹ CFU/day, preferably in an amount from about 10⁶ CFU/day to about 10¹⁰ CFU/day, more preferably in an amount from about 10⁷ CFU/day to about 10⁹ CFU/day.

The term CFU refers to "Colony Forming Units". Quantification of bacteria in a given sample is routinely achieved by counting the total number of colony-forming units (CFUs) grown on an agar plate from serial dilutions, expressed as CFU per gram or mL of the original sample. This yields an estimate of the number of cells present based on a skilled interpretation of the number of colonies on a plate.

A particularly preferred composition of the invention comprises the *Lactobacillus salivarius* PS21603 strain of the invention and maltodextrin. Preferably, the composition comprises the *Lactobacillus salivarius* PS21603 strain of the invention and maltodextrin, wherein the amount of the *Lactobacillus salivarius* PS21603 strain of the invention is from 10⁶ CFU to 10⁸ CFU per gram (g) of composition.

Another particularly preferred composition of the invention comprises the *Lactobacillus salivarius* PS21603 strain of the invention, maltodextrin and an animal feed, such as pig feed. Preferably, said compositions of the invention comprise from about 10⁵ CFU/kg to about 10¹¹ CFU/kg, preferably from about 10⁶ CFU/kg to about 10¹⁰ CFU/kg, more preferably in from about 10⁷ CFU/kg to about 10⁹ CFU/kg, wherein the amounts are expressed as CFU of the strains of the invention per kg of feed.

The composition according to the invention may comprise further probiotics, apart from the *L. salivarius* PS21603 strain of the invention as described above. Probiotics have beneficial effects on the immune system, hence the combination with probiotics will have a superior effect on immune system. Preferably, the further probiotics are selected from the group consisting of *Lactobacillus* and *Bifidobacterium.*

The composition of the present invention may further contain prebiotics. Prebiotics may support the growth of probiotics before they are rendered non-replicating. "Prebiotic" means non-digestible food substances that promote the growth of health beneficial microorganisms and/or probiotics in the intestines. They are not broken down in the stomach and/or upper intestine or absorbed in the GI tract of the person ingesting them, but they are fermented by the gastrointestinal microbiota and/or by probiotics. Preferably, they may be selected from the group consisting of oligosaccharides, optionally containing fructose, galactose, mannose; dietary fibers, in particular soluble fibers, soy fibers; inulin; or mixtures thereof. Preferred prebiotics are fructo-oligosaccharides, galactooligosaccharides, isomalto-oligosaccharides, xylo-oligosaccharides, arabino-xylo oligosaccharides, mannan-oligosaccharides, oligosaccharides of soy, glycosylsucrose, lactosucrose, lactulose, palatinose-oligosaccharides, malto-oligosaccharides, gums and/or hydrolysates thereof, pectins and/or hydrolysates thereof.

### Uses of the strain and composition of the invention

As evidenced by the examples below, the strains and compositions of the invention are safe and have an important beneficial effect. In an *in vivo* animal study, the strain of the invention prevented scours, increased animal weight, reduced fecal *E. coli* concentration, increased the length of the small and large intestine, and improved the ratio of villous height to crypt depth.

Thus, in a further aspects, the present invention relates to a strain or a composition as previously defined for use as a probiotic, as well as a medicament.

In one embodiment, the invention relates to a strain or a composition as previously defined, for use in the prevention of post-weaning diarrhea caused by an *E. coli* infection in a piglet.

As used herein the terms "treat", "treatment", or "treatment of" refers to reducing the potential for a certain disease or disorder, reducing the occurrence of a certain disease or disorder, and/or a reduction in the severity of a certain disease or disorder, preferably, to an extent that the subject no longer suffers discomfort and/or altered function due to it. It also refers to mitigating or decreasing at least one clinical symptom and/or inhibition or delay in the progression of the condition and/or prevention or delay of the onset of a disease or illness. It also means prolonging survival as compared to expected survival if not receiving the treatment.

The term "prevention", "preventing" or "prevent", as used herein, refers to avoiding the appearance of a certain disease or disorder. The prevention can be complete (e.g. the total absence of a disease). The prevention can also be partial, such that for example the occurrence of a disease in a subject is less than that which would have occurred without the administration of the combination or composition of the present invention. Prevention also refers to reduced susceptibility to a clinical condition. The prevention also includes reducing the risk of suffering the disease.

The strain and compositions of the invention also improve performance of an animal. The term "performance" refers to the productivity of an animal, such as a pig, measured by one or more of the following parameters: scours, mortality, number of animals born, number of animals born alive, litter birth weight, and immune system function. An improved performance refers to an improvement in at least one of the previously listed parameters.

Accordingly, a further disclosure falling outside the scope of the present invention relates to a strain or a composition as previously defined for use in improving performance of a mammal animal, preferably a pig.

The strains and composition for use in the present invention described above are preferably administered enterically, more preferably orally. The strain and composition for use according to the invention may be administered in a single daily dose or multiple doses per day, such as at least 2 doses per day, at least 3 doses per day, at least 4 doses per day. Preferably, the strain and composition according to the invention are administered 1, 2 or 3 doses per day. The person skilled in the art will be able to adjust the therapeutically effective dose and/or the prophylactic effective dose appropriately. The precise dose depends on a number of factors such as the mammal's health and weight. Preferably the strain and composition of the invention are administered in an amount that provides a dose of the strain of 10⁵ CFU/day to about 10¹¹ CFU/day, preferably in an amount from about 10⁶ CFU/day to about 10¹⁰ CFU/day, more preferably in an amount from about 10⁷ CFU/day to about 10⁹ CFU/day to the mammal receiving said strain or composition.

In an embodiment of the invention the strain or composition of the invention is for use in a pig. In a preferred embodiment, the pig is a piglet or a weaner, even more preferably a weaner.

Preferably, the strain and composition of the invention is administered for about 2 to about 6 weeks, more preferably for about 3 to about 5 weeks, still more preferably for about 4 weeks (or about 1 month). Preferably, the strain and composition of the invention is administered during the weaning period, more preferably for about 2 to about 6 weeks after weaning, even more preferably for about 3 to about 5 weeks after weaning, still more preferably about 4 weeks after weaning (or about 1 month after weaning).

### EXAMPLES

### Example 1: Identification and characterization of the strain PS21603

### 1.1. Isolation of Lactobacillus salivarius PS21603 and identification (species and strain)

*Lactobacillus salivarius* PS21603 was isolated from the intestine of a wild boar. After the necropsy of the animal, gut content was recovered, diluted and plated in MRS (supplemented with cysteine (MRScys) medium. The incubation was done in anaerobic conditions, at 37°C for 48h.

The strain was identified at the species level as *Lactobacillus salivarius* by PCR amplification of a section of a 16S rRNA gene variable region using primers plb16 (5'-AGAGTTTGATCCTGGCTCAG-3', SEQ ID NO: 1) and mlb16 (5'-GGCTGCTGGCACGTAGTTAG-3', SEQ ID NO: 2) [Kullen et al., Journal of Applied Microbiology, 2000, 89, 511-518]. PCR conditions were as follows: 96 °C for 30 s, 50 °C for 30 s and 72 °C for 45 s (35 cycles) and a final extension at 72 °C for 4 min. Amplified fragments were purified using the NucleoSpin Extract II (Macherey-Nagel Gmb; Düren, Germany) and sequenced using the primers cited above on an ABI 377A automated sequencer (Applied Biosystems, Foster City, USA). The sequences were compared with those deposited in the EMBL database using BLAST algorithm (http://www.ncbi.nlm.nih.gov/BLAST)

The fragment sequenced from *L. salivarius* PS2160316S rRNA gene has SEQ ID NO:3, which is reproduced below.

TTAAACGTCATCGATGACAGTTACTCTCACTCGTGTTCTTCTCTAACAACAG
AGTTTTACGATCCGAAGACCTTCTTCACTCACGCGGCGTTGCTCCATCAGAC

TTGCGTCCATTGTGGAAGATTCCCTACTGCTGCCTCCCGTAGGAGTTTGGGC
CGTGTCTCAGTCCCAATGTGGCCGATCAACCTCTCAGTTCGGCTACGTATCA
TCACCTTGGTAGGCCGTTACCCCACCAACTAGTTAATACGCCGCGGGTCCAT
CTAAAAGCGATAGCAGAACCATCTTTCATCTAAGGATCATGCGATCCTTAG
AGATATACGGTATTAGCACCTGTTTCCAAGTGTTATCCCCTTCTTTTAGGCA
GGTTACCCACGTGTTACTCACCCGTCCGCCACTCAACTTCTTACGGTGAATG
CAAGCATTCGGTGTAAGAAAGTTTCGTTCGACTTGCATGTATTAGGCACGCC
GCCAGCGTTCGTCCTGAGCCAGGATCCAAACTCAAAAA

The identification was confirmed by Matrix Assisted Laser Desorption Ionization-Time of Flight (MALDI-TOF) mass spectrometry using a Vitek-MS^{™} instrument (BioMérieux, Marcy l'Etoile, France) in the facilities of Probisearch (Tres Cantos, Spain). Briefly, a portion of a bacterial colony (~1 µL) was directly spotted onto a MALDI sample plate. Then, it was overlaid with 1 µL of a saturated solution of α-cyano-4-hydroxycinnamic acid in acetonitrile (28%), and allowed to dry at room temperature. A mean spectrum was constructed with at least 50 m/z spectra profiles and used for the identification by comparison with the spectra contained in the Myla database (Biomerieux). Identification was defined as a 99% match to the species-specific m/z values in the database.

The strain could be differentiated from other *L. salivarius* strains of our own collection by genotyping by randomly amplified polymorphic DNA (RAPD), pulsed-field gel electrophoresis (PFGE) analyses and the complete genome sequence.

The complete genome of the strain *L. salivarius* PS21603 has been sequenced and analyzed.

### 1. 2. Antibiogram

Following plate microdilution method referred by EFSA, the MIC of the 8 antibiotics included in this study was determined on *L. salivarius* PS21603 cultures grown overnight in lactic acid bacteria susceptibility test medium (LSM) [Klare et al., Journal of Antimicrobial Chemotherapy, 2005, 59, 900-912] and diluted to obtain a density corresponding to McFarland standard 1 (spectrophotometric equivalent ~ 3 x 10⁸ CFU/mL). The suspension was further adjusted to 3 x 10⁵ CFU/ml with LSM, and 100 µl were inoculated to wells of microtiter VetMIC plates for lactic acid bacteria (National Veterinary Institute of Sweden, Uppsala, Sweden). The plates were incubated at 37 °C for 48 h and the MIC was defined as the lowest concentration at which no growth was observed.

Results from the different antibiotic susceptibility tests were interpreted according to the cut-off levels proposed by the European Food Safety Authority (EFSA, 2017). According to these values, the lactobacillus was sensitive to all the antibiotics tested in this study with the exception of kanamycin, an aminoglycoside antibiotic (Table 1). Bacteria of the genus *Lactobacillus,* like other anaerobic bacteria, tend to be intrinsically resistant to aminoglycosides. This fact implies a certain level of resistance to this group of antibiotics, among which we find gentamicin, kanamycin and streptomycin (EFSA BIOHAZ Panel, 2017, Ricci et al., EFSA Journal, 2017, 15(3), 4664, 177 pp., doi 10.2903/j.efsa.2017.4664). Resistance to these antibiotics has been registered by several authors (Charteris et al., J Food Prot, 1998, 61(12), 1636-1643; Danielsen and Wind, Int J Food Microbiol, 2003, 82(1), 1-11; Zhou et al., Int J Food Microbiol, 2005, 98(2), 211-217; Korhonen et al., J Appl Microbiol, 2007, 103(6), 2496-2503; Guimonde et al., Front Microbiol, 2013, 4, 202, doi: 10.3389/fmicb.2013.00202; Jaimee and Halami, Appl Microbiol Biotechnol, 2016, 100(3), 1137-1151, doi: 10.1007/s00253-015-7184-y), suggesting the existence of an intrinsic resistance. In general, the lack of oxidative metabolism and a low membrane potential (Δψ)*,* which imply a low rate of electron transport and a decrease in the internalization rate of antibiotic molecules. These membrane characteristics are responsible for the resistance in the genus *Lactobacillus* to the aminoglycosides (Bryan and Kwan, J Antimicrob Chemother, 1981, 8 Suppl D, 1-8; Taber et al., Microbiol Rev, 1987, 51(4), 439-457; Schlessinger, Clin Microbiol Rev, 1988, 1, 54-59, https://doi.org/10.1128/CMR.1.1.54; Elkins and Mullis, Appl Environ Microbiol, 2004, 70(12), 7200-7209, doi: 10.1128/AEM.70.12.7200-7209.2004; Steyger, The Volta Review, 2005, 105(3) (monograph), 299-324; Jana and Deb, Appl Microbiol Biotechnol, 2006, 70, 140-150, doi 10.1007/s00253-005-0279-0; Ramírez and Tolmasky, Drug Resist Updat, 2010, 13(6), 151-171, doi:10.1016/j.drup.2010.08.003; Abede et al., Europ. J. Appl. Sci., 2016, 8(5), 301-310, doi: 10.5829/idosi.ejas.2016.8.5.1143).

In addition to the antibiotic sensitivity pattern analysis by the plate microdilution technique, the complete genome sequence of the strain was analyzed with The Comprehensive Antibiotic Resistance Database (CARD), a bioinformatic tool recommended by the EFSA, not finding genes related to any mechanism of resistance to known antibiotics.

**Table 1. Minimum inhibitory concentrations (MICs), and EFSA cut-off values (µg/ml), of antibiotics against L. salivarius PS21603.**

| **Antibiotic** | **Cut-off values*** | **MICs (*L. salivarius* PS21603)** |
|---|---|---|
| Ampicillin | 4 | 1 |
| Gentamicin | 16 | 4 |
| Kanamycin | 64 | 128 |
| Streptomycin | 64 | 64 |
| Erythromycin | 1 | 0.12 |
| Clindamycin | 1 | 0.12 |
| Tetracyclin | 8 | 0.5 |
| Chloramphenicol | 4 | 1 |
| *EFSA (2017); Ricci et al., EFSA Journal, 2017, 15(3), 4664; FEEDAP panel EFSA 2017 | | |

### 1.3. L. salivarius P21603 growth

MRS broth was inoculated with *L. salivarius* PS21603 strain in a 1% proportion. Each 2 h, during the next 24 h, OD₆₀₀, pH and bacterial counts were registered, in duplicate. Briefly, a PS21603 overnight culture in MRS (Oxoid) was used to inoculate 100 mL fresh MRS broth in a 1% proportion, establishing an OD₆₀₀ initial point of 0.0. The broth with the inoculum was mixed in a magnetic stirrer before filling 2 24-well plates. Well plates were incubated at 37ºC and the OD at 600 nm was determined every 2 hours for 24 h using a spectrophotometer (Anthos Zenyth 200rt microplate reader). In parallel, every 2 hours, 2 wells were recovered to measure pH and to count cell numbers. pH measurement was performed in a 3520 pH Meter (Jenway). To perform bacterial counts, each sample was 10-fold diluted, plated in MRS agar plates and incubated at 37°C for 48h in aerobic conditions. After incubation, colony forming units (CFU) were counted and bacterial concentration was calculated. Results were represented as curves, showing the growth evolution of the strain. In addition, math models were calculated to predict CFU/mL and pH based on OD₆₀₀ values in the Curve Expert Software (Hyams Development).

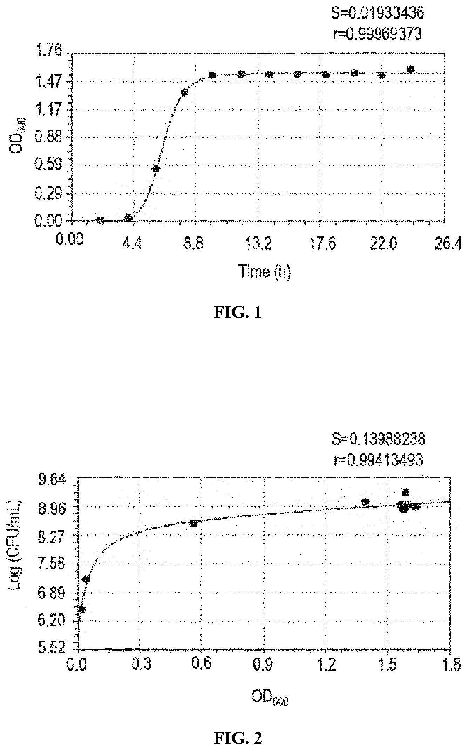

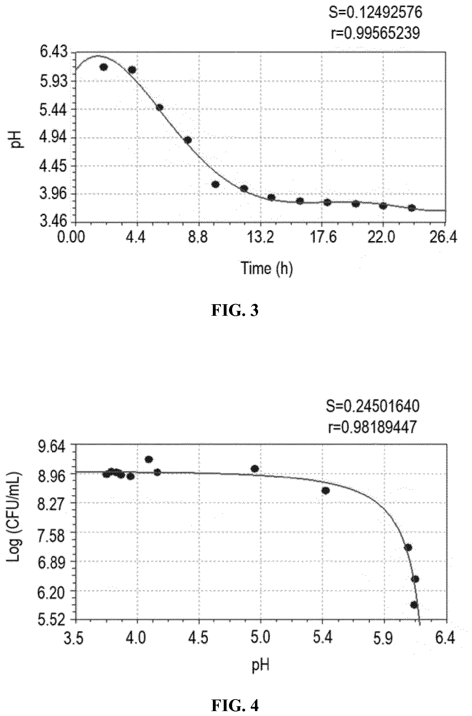

As shown in Figures 1 and 2, *L. salivarius* PS21603 is able to reach the stationary phase after incubation at 37°C (± 1 °C) in aerobiosis in 8 hours, reaching values at this point of 10⁹ CFU/mL, making it a good strain for industrial production, reaching an acceptable biomass soon. In addition, it is able to rapidly acidify the medium in which it grows, reaching pH values of 4 after 8 hours of growth (Figures 3-4).

### 1.4. Resistance to acidic pH

MRS broth tubes, adjusted to different pH (2, 2.5, 3, 3.5 and 4) with HCl in a 3520 pH Meter (Jenway), were inoculated with *L. salivarius* PS21603 strain in 1% proportion. During the next 24h, OD₆₀₀ was registered in Anthos Zenyth 200rt microplate reader, in triplicate. The resulting curves show the growth of the strain and its tolerance to acidity.

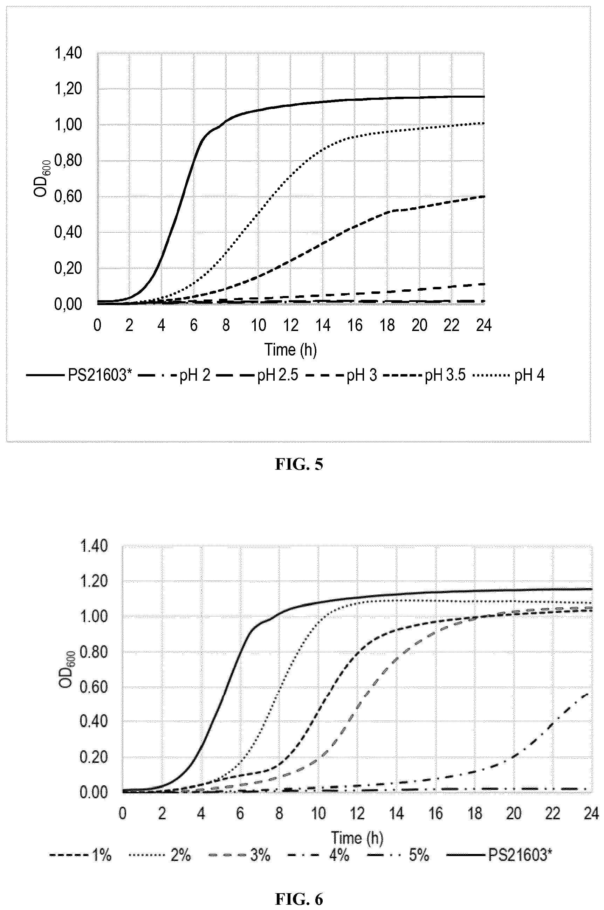

As shown in Figure 5, *Lactobacillus salivarius* strain PS21603 is able to grow at pH above 3.5, albeit at a reduced rate. At a pH of 3, there is hardly any growth and below it growth is inhibited. Despite this, *Lactobacillus salivarius* PS21603 is able to survive for 2 h at low pH (pH 2), so it is expected that it has a high capacity to survive its passage through the stomach in an *in vivo* model, maintaining a number of viable cells enough to exert their effect on the animal's intestine.

### 1.5. Resistance to osmotic changes

MRS broth tubes, adjusted to NaCl concentrations (1, 2, 3, 4 and 5%), were inoculated with *L. salivarius* PS21603 strain in 1% proportion. During the next 24h, OD₆₀₀ was registered in Anthos Zenyth 200rt microplate reader, in triplicate. The resulting curves show the growth of the strain and its tolerance to different osmotic pressure.

*Lactobacillus salivarius* strain PS21603 presents a high tolerance to osmotic stress, represented in this case by the concentration of NaCl. Said strain is able to grow up to a concentration of 4% NaCl, which corresponds to high osmotic stress (Figure 6). This data is applicable to lyophilization-rehydration cycles, which are a necessary step for production on an industrial scale. In conclusion, the strain PS21603 presents *in vitro a* high capacity to support the lyophilization-rehydration process.

### 1. 6. Effect on E. coli

The ability of the *L. salivarius* PS21603 strain to inhibit *E. coli* was investigated. PS21603 strain and *E. coli* CECT501 strain were incubated separately in MRS and BHI broth, respectively, under aerobiosis conditions until OD₆₀₀ = 0.6. Aliquots of the PS21603 strain culture were mixed with equal volumes of each of the pathogen cultures in BHI and MRS, and incubation was carried out in aerobic conditions at 37°C. Samples from the mixed cultures were plated at the beginning and at the end of the incubation process on the appropriate media. Plates were incubated at 37°C for 48h and colony-forming units (CFU) counted. The experiment was performed in triplicate.

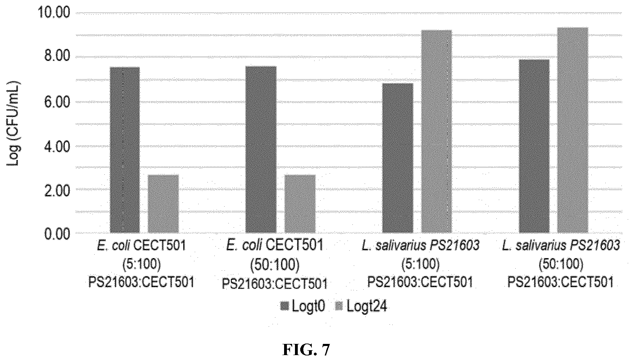

In the 24-hour co-culture with the *E. coli* strain having accession number CECT501 (given by the Colección Española de Cultivos Tipo), with serotype O149: K91, K88a, c: H10, the presence of *L. salivarius* PS21603 induces a reduction of 5 logarithmic units of the pathogen (Figure 7). This is a clear inhibition of the growth of this *E. coli* strain, whether starting at the same concentration or with a 10-fold lower concentration, showing the utility of the *L. salivarius* PS21603 strain in enhancing the resistance to *E. coli* infection which is the main cause of post-weaning diarrhea [Huang et al., *Asian-Aust J Anim Sci,* 2004, 17(3), 401-409; Dowarah et al., *Livestock Science,* 2017, 195, 74-79].

### Example 2. In vivo study in piglets

### 2.1. Study design

A 28-day study was carried out in piglets after weaning on a farm owned by PigCHAMP, a world leader in pig research. This farm, located in the municipality of Aguilafuente (Segovia, Spain), is a commercial farm that is adapted for animal experimentation. The study was authorized by the Junta de Castilla y León (Spain).

The study included a total of 384 animals divided into three study arms, two supplemented with the strain *L. salivarius* PS21603 at different doses, and a third control arm, which were administered the diet without supplementation. The animals were randomized among the three arms that were homogeneous at the beginning of the study. The total duration of the study was 28 days, so that the animals that started the study at 28 days of age ended at 56 days of age. To arm T1, feed supplemented with *L. salivarius* strain P21603 at a concentration of 10⁹ CFU/kg of feed (dose of 10⁹ CFU/day) was assigned. To arm T2, feed supplemented with *L. salivarius* strain P21603 at a concentration of 10⁷ CFU/Kg of feed (dose of 10⁷ CFU/day) was assigned. To arm T3, control diet, without supplementation was assigned. Each of the arms was divided into pens, assigned randomly within the farm rooms. The *L. salivarius* strain P21603 was administered lyophilized without encapsulating and vehiculated in maltodextrin to give volume to the preparation.

To monitor the growth and fattening of the animals, the production data of each animal was recorded at the beginning (day 0), at day 7, at day 14 and at the end of the study (day 28). During the intervention period, samples of feces and blood of 16 animals per arm were collected at the beginning and at the end of the study. Once the study was finished, 6 animals of each group were sacrificed for the macroscopic and histological analysis of the small and large intestine. Samples of feed were taken from each batch prepared for each group of animals in order to know the actual concentration administered of the strain.

### 2.2. Results

### 2.2.1. Growth and weight gain (animal performance)

All the animals belonging to the batch used in the study were exceptional in terms of general health and, specifically, intestinal health. There were no cases of diarrhea in any of the arms and all grew above the mean values observed in the history of the farm, with no differences in weight gain with respect to the group assigned to the animals (Table 2). This makes the results obtained in this trial especially relevant, since we consider that all the findings are due to the effect of the strain in a perfectly healthy population, showing the safety and tolerance of the strain.

### 2.2.2. Presence of E. coli in feces

The feces' samples collected at the beginning (WO) and at the end of the study (W4) were plated in McK, a solid medium specific for the growth of enterobacteria, to assess *E. coli* counts.

The animals that received the dose of 10⁹ CFU/day presented a significant reduction of the *E. coli* counts in feces when comparing with the control group. Those animals that received the dose 10⁷ CFU/day showed a tendency of the *E*. *coli* reduction (Table 3). No bacteria of the genus *Salmonella* were detected in any of the samples.

### 2.2.3. Immune profile

In the blood samples, the concentration of 9 cytokines (IL-1β, IL-4, IL-6, IL-8, IL-10, IL12, INFα, INFγ and TNFα) was studied to determine if there was a systemic effect on the immune system of the strain under study (Table 3). The changes observed in this analysis corresponded to the maturation of the immune system of the piglets. No effect of the strain on these cytokines was observed at a systemic level. No differences between associated with the administration of the strain were found.

### 2.2.4. Anatomy and histology of the intestine

The macroscopic analysis performed at the necropsy of the sacrificed animals showed an enlargement of the small and large intestine in the study groups which received diet supplemented with the strain *L. salivarius* PS21603 (Table 4). In addition, this effect was dose-dependent. At the histological level (Table 5), in the animals treated with the dose 10⁷ CFU/day, an improvement in the villus height/crypt depth ratio (VH/CD) was observed after the ingestion of the strain for 28 days. A higher VH/CD ratio is associated with a better intestinal capacity and better resistance to diarrhea at weaning [Dowarah et al., Livestock Science, 2017, 195, 74-79].

**Table 2. Growth performance results**

| | **BW0 BW7 BW14 BW28 Hom_BW0** | **Hom_BW7 Hom_BW14 Hom_BW28 ADG0_7 ADG7_14** | **ADG0_14 ADG14_28 ADG0_28** |
|---|---|---|---|
| **T1** | 8.73 9.89 11.54 16.41 93.31 92.37 91.3 2 | 88.96 144.0 | 110.7 187.6 374.1 274.7 |
| **T2** | 8.74 9.84 11.51 16.32 92.81 91.72 90.9 8 | 88.56 137.4 | 110.2 184.3 370.6 270.7 |
| **T3** | 8.75 9.82 11.59 16.33 93.01 91.92 91.26 | 89.54 134.8 | 118.2 190.1 364.8 271.0 |
| ***SEM*** | 0.276 0.058 0.099 0.194 0.442 0.591 0. 742 | 0.762 7.226 5.120 6.494 12.155 6.891 | |
| ***P-value*** | 0.999 0.656 0.842 0.939 0.728 0.724 | 0.943 0.660 0.653 0.467 0.822 0.861 0.899 | |

| | | | |
|---|---|---|---|
| T1: Treatment 10⁹ CFU/day, T2: Treatment 10⁷ CFU/day, T3: control, SEM: Standard error of the mean, BW#: body weight (g), # is the number of days after start of the study; Horn: Homogeneity; ADG#1_#2: Average Daily Gain (g), wherein #1 and #2 indicate the days after start of the study that are compared. | | | |

**Table 3. E. coli counts and blood cytokines levels.**

| n | **T1 (N=16) T2 (N** | | | | | **=16) T3 (N=16)** | | | | | | | | | | **p-value** | |
|---|---|---|---|---|---|---|---|---|---|---|---|---|---|---|---|---|---|
| | **W0 W4** | | | | **KW p-value** | **W0 W4** | | | | **KW p-value** | **W0 W4** | | | | **KW p-value** | | |
| | | Median (IQR) | n | Median (IQR) | | n | Median (IQR) | n | Median (IQR) | | n | Median (IQR) | n | Median (IQR) | | W0 | W4 |
| ***E. coli*** | 1 6 | 1.00E+05 (4.38E+04, 6.63E+05) | 16 | 3.75E+05 (8.88E+04, 1.18E+06) | 0.509 | 16 | 3.25E+05 (1.00E+04, 1.13E+06) | 1 6 | 2.00E+06 (6.25E+05, 4.29E+06) | **0.022** | 16 | 3.03E+05 (7.88E+04, 4.25E+06) | 16 | 3.00E+06 (7.88E+05, 9.25E+06) | **0.025** | 0.522 | **0.006** |
| **IL-1β** | 2 | 51.67 (46.92, 56.43) | 0 | NA (NA, NA) | --- | 1 | 78.66 (78.66, 78.66) | 2 | 5.76 (4.79, 6.72) | 0.221 | 1 | 1.58 (1.58, 1.58) | 0 | NA (NA, NA) | --- | 0.259 | --- |
| **IL-4** | 3 | 8.62 (6.49, 8.83) | 5 | 2.3 (2.16, 2.30) | 0.177 | 3 | 5.46 (4.04, 23.98) | 1 | 2.21 (2.21, 2.21) | 0.180 | 2 | 16.99 (9.65, 24.34) | 3 | 4.42 (3.36, 8.85) | 0.767 | 0.946 | 0.377 |
| **IL-6** | 1 | 209.93 | 3 | 3.1 | 0.180 | 1 | 49.3 | 2 | 7.23 (4.53, 9.93) | 0.221 | 0 | NA (NA. NA) | 3 | 10.42 (6.61. 12.05) | --- | 0.317 | 0.895 |
| | | (209.93, 209.93) | | (2.26, 31.09) | | | (49.30, 49.30) | | | | | | | | | | |
| **IL-8** | 1 5 | 60.27 (27.98, 281.98) | 15 | 373.71 (231.69, 472.41) | **0.001** | 14 | 171.35 (49.32, 282.92) | 1 4 | 349.85 (272.39, 692.78) | **0.002** | 14 | 152.31 (22.86, 314.97) | 14 | 370.87 (335.12, 625.56) | **0.007** | 0.763 | 0.955 |
| **IL-10** | 1 | 113.98 (113.98, 113.98) | 1 | 153.26 (153.26, 153.26) | --- | 2 | 126.45 (88.85, 164.05) | 0 | NA (NA, NA) | --- | 0 | NA (NA, NA) | 0 | NA (NA. NA) | --- | 1.000 | --- |
| **IL-12p40** | 1 3 | 754.85 (463.21, 952.96) | 13 | 1.080.18 (665.56, 1.927.42) | 0.161 | 13 | 602.45 (422.07, 2.110.76) | 1 4 | 1.076.93 (655.29, 1.785.77) | 0.207 | 12 | 662.24 (469.99, 938.73) | 15 | 1.050.11 (552.20, 1.396.81) | 0.107 | 0.957 | 0.916 |
| **INFα** | 1 3 | 7.28 (5.41, 20.90) | 9 | 3.14 (1.15, 7.01) | 0.133 | 15 | 20.05 (5.59, 72.43) | 1 1 | 1.75 (0.56, 5.85) | 0.007 | 13 | 12.85 (4.70, 36.33) | 12 | 2.74 (0.89, 9.40) | 0.103 | 0.424 | --- |
| **INFγ** | 1 | 53.42 (53.42, 53.42) | 0 | NA (NA, NA) | --- | 2 | 239 (127.45, 350.54) | 0 | NA (NA, NA) | --- | 0 | NA (NA, NA) | 0 | NA (NA, NA) | --- | 1.000 | --- |
| **TNFα** | 1 | 191.11 (191.11, 191.11) | 0 | NA (NA, NA) | --- | 1 | 434.04 (434.04, 434.04) | 0 | NA (NA, NA) | --- | 0 | NA (NA, NA) | 0 | NA (NA, NA) | --- | 0.317 | 0.778 |

| | | | | | | | | | | | | | | | | | |
|---|---|---|---|---|---|---|---|---|---|---|---|---|---|---|---|---|---|
| T1: Treatment 10⁹ CFU/day, T2: Treatment 10⁷ CFU/day, T3: control, W0: initial week; W4: final week, KW: Kruskal-Wallis test, NA: not available. | | | | | | | | | | | | | | | | | |

**Table 4. Intestine anatomy results**

| | **LENGTH (m)** | |
|---|---|---|
| | **SI LI** | |
| **T1** | 14.612 | 3.000 |
| **T2** | 13.711 | 2.748 |
| **T3** | 13.360 | 2.519 |
| ***SEM*** | 0.296 | 0.107 |
| ***P-value*** | 0.034 | 0.029 |

| | | |
|---|---|---|
| T1: Treatment 10⁹ CFU/day, T2: Treatment 10⁷ CFU/day, T3: control, SI: Small intestine, LI: Large intestine, SEM: Standard error of the mean. | | |

**Table 5. Intestine histology morphometry**

| ***JEJUNUM*** | | | | | | | | | | | |
|---|---|---|---|---|---|---|---|---|---|---|---|
| | **VH** | **CD** | **VH:CD** | **ratio** | **IEL** | **IEL/100** | **µm** | **GC** | **GC/100um** | **MITOSES** | **MITOSES/100 µm** |
| **T1** | 616.7 | 209.8 | 3.105 | 16.98 2. | | | 81 1 | 1.25 | 3.54 | 0.573 | 0.260 |
| **T2** | 636.5 | 201.2 | 3.337 | 15.32 2. | | | 44 | 10.51 | 1.70 | 0.800 | 0.398 |
| **T3** | 558.3 | 218.3 | 2.670 | 16.44 | | | 2.92 | 11.75 | 2.10 | 0.756 | 0.348 |
| ***SEM*** | 35.520 | 8.836 | 0.158 | 0.918 | 0.185 | 0.430 | 1.090 | 0.152 | 0.066 | | |
| ***P-value*** | 0.280 | 0.411 | 0.027 | 0.390 | 0.152 | 0.133 | 0.416 | 0.496 | 0.291 | | |

| ***ILEUM*** | | | | | | | | | | | |
|---|---|---|---|---|---|---|---|---|---|---|---|
| | **VH** | **CD** | **VH:CD** | **ratio** | **IEL** | **IEL/100** | **µm** | **GC** | **GC/100um** | **MITOSES** | **MITOSES/100 µm** |
| **T1** | 404.0 | 217.0 | 1.938 | 13.41 | | | 3.36 | 5.62 | 1.41 | 0.487 | 0.228 |
| **T2** | 377.2 | 173.0 | 2.477 | 14.96 | | | 4.20 | 5.15 | 1.40 | 0.490 | 0.283 |
| **T3** | 349.3 | 207.2 | 1.822 | 17.68 | | | 5.56 | 3.74 | 1.11 | 0.513 | 0.260 |
| ***SEM*** | 36.096 | 12.412 | 0.313 | 1.848 | 0.718 | 0.672 | 0.196 | 0.117 | 0.061 | | |
| ***P-value*** | 0.576 | 0.058 | 0.316 | 0.151 | 0.125 | 0.156 | 0.491 | 0.985 | 0.818 | | |

| | | | | | | | | | | | |
|---|---|---|---|---|---|---|---|---|---|---|---|
| T1: Treatment 10⁹ CFU/day, T2: Treatment 10⁷ CFU/day, T3: control, VH: villus height, CD: crypt depth, IEL: intra-epithelial lymphocytes, GC: Goblet cells, SEM: Standard error of the mean | | | | | | | | | | | |

### 2.2.5. Concentration of the product in the feed

Feed samples were diluted 10-times in peptone water (Oxoid) and digested in a masticator. Serial 10-fold dilutions were prepared in peptone water and plated in MRScys agar plates (Oxoid). Plates were incubated at 37°C, in anaerobiosis, for 48h. After the incubation, CFU were counted and bacterial concentration per feed gram calculated considering the dilution in which colonies were counted.

As for the feed samples, strain *L. salivarius* PS21603 has been recovered at the concentration that was estimated to comply with the doses of 10⁷ and 10⁹ CFU/day, so that the animals were correctly supplemented during the study period.

### 2.2.6. Intestinal microbiome

The analysis of the sequencing of the fecal microbiome showed that the strain is extremely selective against *E. coli.* The administration of *L. salivarius* strain PS21603 at a dose of 10⁹ CFU/day significantly reduced the concentration of *E. coli* in the intestine of piglets. This reduction is also appreciable in the group that received the dose of 10⁷ CFU/day, although it is not statistically significant (Table 6).

### 2.2.7. Conclusion

At the end of the study, the *L. salivarius* PS21603 strain was well tolerated by the piglets. Neither growth retarding nor inflammation was detected in treatment groups in comparison to control group. In addition, microbiological and anatomical differences were observed between animals treated with *L. salivarius* P21603 strain with respect to those that were not treated, demonstrating that the treatment does exert an effect on the intestinal microbiota of healthy piglets after weaning.

**Table 6. Microbiome changes expressed in detection frequencies.**

| | | **T1** | | | **T2** | | | **T3** | | |
|---|---|---|---|---|---|---|---|---|---|---|
| | | W0 | W4 | p-value | ≠ W0 | W4 | p-value | *≠* W0 | W4 | p-value |
| Phylum | *Proteobacteria* | 0.107 | 0.051 | 0.087* | 0.078 | 0.054 | NS | 0.079 | 0.058 | NS |
| Family | *Coriobacteriaceae* | 0.015 | 0.035 | NS | 0.016 | 0.038 | 0.036 | 0.023 | 0.017 | NS |
| | *Erysipelotrichaceae* | 0.019 | 0.025 | NS | 0.014 | 0.038 | 0.005 | 0.017 | 0.017 | NS |
| | *Coprobacillaceae* | 0.002 | 0.048 | 0.017 | 0.006 | 0.057 | 0.005 | 0.007 | 0.007 | NS |
| | *Flexibacteraceae* | 0.003 | 0.009 | NS | 0.004 | 0.008 | NS | 0.006 | 0.014 | 0.031 |
| | *Streptococcaceae* | 0.005 | 0.002 | 0.041 | 0.002 | 0.002 | NS | 0.003 | 0.002 | NS |
| | *Turicibacteraceae* | 0.004 | 0.000 | 0.000 | 0.001 | 0.000 | NS | 0.001 | 0.000 | NS |
| | *Thermomonosporaceae* | 0.001 | 0.002 | NS | 0.001 | 0.001 | NS | 0.001 | 0.001 | NS |
| | *Geobacteraceae* | 0.000 | 0.001 | 0.009 | 0.000 | 0.001 | 0.000 | 0.000 | 0.000 | NS |
| | *Saprospiraceae* | 0.000 | 0.001 | 0.000 | 0.000 | 0.001 | 0.000 | 0.000 | 0.001 | NS |
| Genus | *Catenibacterium* | 0.001 | 0.050 | 0.014 | 0.003 | 0.061 | 0.002 | 0.007 | 0.007 | NS |
| | *Escherichia* | 0.049 | 0.003 | 0.017 | 0.023 | 0.004 | NS | 0.025 | 0.012 | NS |
| | *Slackia* | 0.008 | 0.021 | 0.015 | 0.009 | 0.026 | 0.000 | 0.010 | 0.012 | NS |
| | *Megasphaera* | 0.005 | 0.019 | NS | 0.001 | 0.011 | NS | 0.002 | 0.031 | 0.000 |
| | *Streptococcus* | 0.006 | 0.002 | 0.049 | 0.002 | 0.002 | NS | 0.003 | 0.002 | NS |
| | *Turicibacter* | 0.004 | 0.000 | 0.000 | 0.001 | 0.000 | 0.000 | 0.001 | 0.000 | 0.000 |
| | *Sarcina* | 0.003 | 0.000 | 0.008 | 0.001 | 0.000 | NS | 0.001 | 0.000 | NS |

| | | | | | | | | | | |
|---|---|---|---|---|---|---|---|---|---|---|
| T1: Treatment 10⁹ CFU/day, T2: Treatment 10⁷ CFU/day, T3: control, W0: initial week; W4: final week, NS: non-significant. ^{‡}p-value from HSD de Tukey test. * 90% signification level | | | | | | | | | | |

## Claims

1. A strain of *Lactobacillus salivarius* PS21603 (CECT 9673).

2. The strain as defined in claim 1, wherein the strain is alive.

3. A composition comprising a strain as defined in any of claims 1 to 2.

4. The composition according to claim 3, which further comprises a veterinary or pharmaceutically acceptable excipient.

5. The composition according to claim 4, wherein the veterinary or pharmaceutically acceptable excipient is maltodextrin.

6. The composition according to any of claims 3 to 5, which further comprises a foodstuff.

7. The composition according to claim 6, wherein the foodstuff is an animal feed.

8. A strain as defined in any of claims 1 to 2 or a composition as defined in any of claims 3 to 7, for use as a probiotic.

9. A strain as defined in any of claims 1 to 2 or a composition as defined in any of claims 3 to 7, for use as a medicament.

10. A strain as defined in any of claims 1 to 2 or a composition as defined in any of claims 3 to 7, for use in the prevention of post-weaning diarrhea caused by an *E. Coli* infection in a piglet.

## Patentansprüche

1. Stamm von *Lactobacillus salivarius* PS21603 (CECT 9673).

2. Stamm nach Anspruch 1, wobei der Stamm lebend ist.

3. Zusammensetzung, die einen Stamm wie in einem der Ansprüche 1 bis 2 definiert aufweist.

4. Zusammensetzung nach Anspruch 3, die ferner einen veterinärmedizinischen oder pharmazeutisch verträglichen Hilfsstoff aufweist.

5. Zusammensetzung nach Anspruch 4, wobei der veterinärmedizinische oder pharmazeutisch verträgliche Hilfsstoff Maltodextrin ist.

6. Zusammensetzung nach einem der Ansprüche 3 bis 5, die außerdem ein Nahrungsmittel aufweist.

7. Zusammensetzung nach Anspruch 6, wobei das Nahrungsmittel ein Tierfutter ist.

8. Stamm nach einem der Ansprüche 1 bis 2, oder Zusammensetzung nach einem der Ansprüche 3 bis 7, zur Verwendung als Probiotikum.

9. Stamm nach einem der Ansprüche 1 bis 2, oder Zusammensetzung nach einem der Ansprüche 3 bis 7, zur Verwendung als Arzneimittel.

10. Stamm nach einem der Ansprüche 1 bis 2, oder Zusammensetzung nach einem der Ansprüche 3 bis 7, zur Verwendung bei der Vorbeugung von Durchfall, der nach Abstillen durch eine *E. coli*-Infektion verursacht wird, bei einem Ferkel.

## Revendications

1. Une souche de Lactobacillus salivarius PS21603 (CECT 9673).

2. La souche définie dans la revendication 1, dans laquelle la souche est vivante.

3. Une composition comprenant une souche telle que définie dans l'une quelconque des revendications 1 à 2.

4. La composition selon la revendication 3, qui comprend en outre un excipient vétérinaire ou pharmaceutiquement acceptable.

5. La composition selon la revendication 4, dans laquelle l'excipient vétérinaire ou pharmaceutiquement acceptable est la maltodextrine.

6. La composition selon l'une quelconque des revendications 3 à 5, qui comprend en outre une denrée alimentaire.

7. La composition selon la revendication 6, dans laquelle la denrée alimentaire est un aliment pour animaux.

8. Une souche telle que définie dans l'une quelconque des revendications 1 à 2 ou une composition telle que définie dans l'une quelconque des revendications 3 à 7, pour utilisation comme probiotique.

9. Une souche telle que définie dans l'une quelconque des revendications 1 à 2 ou une composition telle que définie dans l'une quelconque des revendications 3 à 7, pour utilisation en tant que médicament.

10. Une souche telle que définie dans l'une quelconque des revendications 1 à 2 ou une composition telle que définie dans l'une quelconque des revendications 3 à 7, pour utilisation dans la prévention de la diarrhée post-sevrage causée par une infection à *E. Coli* chez un porcelet.
